# EUROPÄISCHE PATENTSCHRIFT

(11) **EP 1 981 418 B1**
(45) Veröffentlichungstag und Bekanntmachung des Hinweises auf die Patenterteilung: **18.03.2015**
(21) Anmeldenummer: 07703148.2
(22) Anmeldetag: 30.01.2007
(51) Int. Cl.: A61B 17/60, A61B 17/72, A61B 17/80, A61B 5/00, A61B 19/00

(54) **FIXATIONSSYSTEM FÜR KNOCHEN MIT EINEM SENSOR- UND TELEMETRIESYSTEM**
FIXATION SYSTEM FOR BONE WITH A SENSOR AND TELEMETRY SYSTEM
SYSTÉME DE FIXATION POUR OS COMPRENANT UN SYSTÉME DE DÉTECTION ET DE TÉLÉMÉTRIE

(30) Priorität: 07.02.2006 DE 102006006341
(43) Veröffentlichungstag der Anmeldung: 22.10.2008
(73) Patentinhaber: Wolter, Dietmar, 22955 Hoisdorf (DE)
(72) Erfinder: CAPANNI, Felix, 21031 Hamburg (DE)
(74) Vertreter: Glawe, Delfs, Moll
(86) Internationale Anmeldenummer: PCT/EP2007/000794
(87) Internationale Veröffentlichungsnummer: WO 2007/090543

(56) Entgegenhaltungen:
- WO-A-00/33752
- WO-A-2005/074821
- DE-A- 19 858 889
- US-A- 6 034 296
- BURNY F ET AL: "CONCEPT, DESIGN AND FABRICATION OF SMART ORTHOPEDIC IMPLANTS" MEDICAL ENGINEERING & PHYSICS, BUTTERWORTH-HEINEMANN, GB, Bd. 22, Nr. 7, September 2000 (2000-09), Seiten 469-479, XP001089871 ISSN: 1350-4533

## Beschreibung

Die Erfindung betrifft ein Fixationssystem für Knochen mit einem Sensor- und Telemetriesystem.

Fixationssysteme werden in der Osteosynthese verwendet, wobei der Verbindungsträger den Bruch überbrückt und über mindestens eine Knochenschraube mit den Knochenfragmenten verbunden wird. Der Verbindungsträger ist beispielsweise eine Knochenplatte, ein Marknagel oder ein Fixateur extern. Dabei ist es vorteilhaft, wenn die Knochenschraube in dem Verbindungsträger dauerhaft winkelstabil verblockt ist.

Besonders vorteilhaft ist eine freie Wählbarkeit der Schraubenrichtung vor der dauerhaft winkelstabilen Verblockung der Knochenschraube im Verbindungsträger. Die winkelstabile Verbindung von Knochenschraube und Verbindungsträger führt zu einem Stabilitätsgewinn der gesamten Montage durch die besondere Art der Übertragung von Kräften und Lasten. Diese Übertragung ist durch die flächenhafte Kraftverteilung charakterisiert. Ferner zeigen biomechanische und klinische Ergebnisse günstigere Heilungsverläufe.

Die Kontrolle der Heilungsvorgänge bei der Osteosynthese ist durch sogenannte bildgebende Verfahren möglich. Es handelt sich hierbei in erster Linie um Röntgenuntersuchungen, Kernspintomographie und Computertomographie. Es ist bekannt, daß diese bildgebenden Verfahren in ihrer Aussagefähigkeit dem wirklichen Heilungsprozeß mehrere Wochen hinterher hinken.

Erforderlich ist, die Fähigkeit des heilenden Gewebes im Hinblick auf die Übertragung von Kräften sicher zu beurteilen, um dadurch auf den Stand des Heilungsprozesses schließen zu können, die Nachbehandlung besser durchführen zu können und um mögliche fehlerhafte Heilungen frühzeitig zu identifizieren.

Fixationssysteme für Knochen wurden schon früher mit Sensorsystemen kombiniert. Hierbei kamen Dehnungsmeßstreifen zum Einsatz, welche mit Kabel verbunden waren, die durch die Haut nach außen geführt wurden. Insbesondere die Infektionsproblematik hat dazu geführt, daß derartige Systeme nur in Ausnahmefällen zum Einsatz kamen.

Seit mehreren Jahren sind Sensorsysteme in klinischer Anwendung, welche mit einem Telemetriesystem zusammenarbeiten, welches die vom Sensorsystem im Körper gewonnenen Meßwerte drahtlos nach außen überträgt. Die Herstellung dieser Systeme ist schwierig, aufwendig und kostenintensiv. Nur in Einzelfällen sind daher diese Systeme - z.B. im Bereich der Wirbelsäule oder des Hüftgelenkes - zum Einsatz gekommen, um Fragen nach der Belastungsfähigkeit, den Kräften und nach der Heilung zu beantworten. Sensor- und Telemetriesystem sind durch Verkleben mit dem Fixationssystem verbunden.

Aufgrund dieser festen Verbindung von Fixations-, Sensor- und Telemetriesystem muß der Operateur frühzeitig die Entscheidung treffen, ob der Patient dieses kombinierte System benötigt.

Erst am Ende der Operation, nachdem die Knochenfixation abgeschlossen ist, ist eine Beurteilung im Hinblick auf den zukünftigen Heilungsverlauf möglich. Viele maßgebliche Faktoren für die Heilung werden durch den operativen Vorgang entscheidend beeinflußt.

Die US-A-6034296 und die WO-A-2005074821 offenbaren Fixationssysteme für Knochen mit einem Verbindungsträger, wenigstens einer in ein Durchgangsloch des Verbindungsträgers einsetzbaren Knochenschraube und einem Sensor- und Telemetriesystem, das auf einer separaten Platte angeordnet ist, die mit dem Verbindungsträger verbindbar ist.

Davon ausgehend liegt der Erfindung die Aufgabe zugrunde, ein Fixationssystem für Knochen mit einem Sensor- und Telemetriesystem zu schaffen, bei dem der Kraftfluß über das Sensorsystem erfolgt.

Die Aufgabe wird durch ein Fixationssystem mit einem Sensor- und Telemetriesystem mit den Merkmalen des Anspruches 1 gelöst. Vorteilhafte Ausgestaltungen des Fixationssystems mit dem Sensor- und Telemetriesystem sind in den Unteransprüchen angegeben.

Gemäß Anspruch 1 hat das Fixationssystem für Knochen einen Verbindungsträger, wenigstens eine in ein Durchgangsloch des Verbindungsträgers einsetzbare Knochenschraube und ein Sensor- und Telemetriesystem, wobei das Sensor- und Telemetriesystem auf einer separaten Platte angeordnet ist, die mit dem Verbindungsträger verbindbar ist, dadurch gekennzeichnet, daß Verbindungsträger und separate Platte Einrichtungen zum Ausbilden mindestens einer Nut-Feder-Verbindung zwischen Verbindungsträger und Platte aufweisen.

Dadurch, daß bei dem erfindungsgemäßen Fixationssystem das Sensor- und Telemetriesystem auf einer separaten Platte angeordnet ist, die mit dem Verbindungsträger verbindbar ist, kann der Operateur am Schluß der Operation entscheiden, ob das Fixationssystem mit dem Sensor- und Telemetriesystem ausgestattet werden muß. Falls dies nicht erforderlich ist, läßt er das Sensor- und Telemetriesystem weg und setzt nur das Fixationssystem ein. Hierdurch werden Aufwand und Kosten eingespart. Dem Operateur wird die Entscheidung über den Einsatz dieses kombinierten Systems erleichtert. Benötigt jedoch der Patient das kombinierte System, so kann der Operateur die Platte mit dem Sensor- und Telemetriesystem einfach mit dem Fixationssystem verbinden.

Die Erfindung ist insbesondere für Fixationssysteme nutzbar, bei denen mindestens eine Knochenschraube eine dauerhaft winkelstabile Verbindung mit dem Träger aufweist. Sie ist z.B. für Fixationssysteme nutzbar, bei denen mindestens eine Knochenschraube in einer definierten Winkelausrichtung in dem Durchgangsloch sitzt. Dabei kann das Durchgangsloch die Knochenschraube in einer definierten Winkelausrichtung führen bzw. mit einem vorgegebenen Gewinde ausgeführt sein, in das die Knochenschraube ausschließlich in einer definierten Ausrichtung einschraubbar ist.

Gemäß einer bevorzugten Ausgestaltung weisen Knochenschraube und Durchgangsloch Einrichtungen zum winkelstabilen Verbinden in wählbarer Winkelausrichtung auf. Diese Einrichtungen sind gemäß einer Ausgestaltung Einrichtungen zum Formen eines Gewindes zwischen Knochenschraube und Durchgangsloch. Dies ermöglicht es, auch eine in variablen Winkelausrichtungen in den Verbindungsträger einsetzbare Knochenschraube dauerhaft winkelstabil mit dem Verbindungsträger zu verbinden.

Die Einrichtungen zum Formen eines Gewindes umfassen z.B. ein Gewinde an der Knochenschraube und ein gewindeloses Durchgangsloch, in das die Knochenschraube unter wählbarem Winkel unter Formung eines Innengewindes eindrehbar ist. Gemäß einer Ausgestaltung weisen hierbei die Materialien von Knochenschraube und der Innenwand des Durchgangsloches oder des Verbindungsträgers unterschiedliche Härtegrade auf. Z.B. wird Reintitan für die Knochenschraube und eine Titanlegierung für den Verbindungsträger verwendet oder umgekehrt. Weitere mögliche Ausgestaltungen der Einrichtung zum Formen eines Gewindes sind in der DE 43 43 117 C2 beschrieben.

Die Erfindung ermöglicht den Einsatz verschiedener Sensorsysteme. Das Sensorsystem mißt beispielsweise chemische Parameter oder die Temperatur oder das Fixationssystem belastende Kräfte oder die Verformung des Fixationssystems. Gemäß einer Ausgestaltung umfaßt das Sensorsystem einen Dehnungsmeßstreifen (DMS). Mittels DMS ist die Dehnung des Verbindungsträgers erfaßbar, aus der wiederum die den Verbindungsträger belastenden Kräfte ermittelbar sind.

Insbesondere wenn das Sensorsystem einen Sensor zum Messen von Verformungen oder Kräften umfaßt, ist gemäß einer Ausgestaltung das Sensorsystem so auf der separaten Platte und die separate Platte so auf dem Verbindungsträger montiert, daß der Kraftfluß über den Verbindungsträger und über das Sensorsystem erfolgt, so daß die im Heilungsverlauf abnehmenden Verformungen oder Kräfte gemessen und telemetrisch übertragen werden.

Gemäß einer Ausgestaltung ist das Sensorsystem zum Messen der Verformungen oder Kräfte fest mit der separaten Platte verbunden. Die separate Platte weist gemäß einer Ausgestaltung Einrichtungen zum form- und/oder kraft- und/oder stoffschlüssigen Verbinden von Verbindungsträger und Platte auf. Die Einrichtungen zum Verbinden sind gemäß einer Ausgestaltung auf verschiedenen Seiten des Sensorsystems angeordnet, so daß der Kraftfluß zwischen den Einrichtungen zum Verbinden über das Sensorsystem erfolgt. Durch eine innige Verbindung von separater Platte und Verbindungsträger wird erreicht, daß ein Belastung des Verbindungsträgers entsprechender Kraftfluß über das Sensorsystem erfolgt.

Die Nut-Feder-Verbindung ermöglicht insbesondere eine formschlüssige Verbindung zwischen Verbindungsträger und Platte. Darüber hinaus ist eine kraftschlüssige Verbindung möglich, indem die Feder in die Nut eingepreßt wird. Dabei kann es auch zu einer stoffschlüssigen Verbindung kommen, insbesondere wenn durch Kombination geeigneter Materialien für Feder und Nut eine Verschweißung beim Einpressen der Feder in die Nut stattfindet. Dies ist beispielsweise bei der Wahl einer Titanlegierung für die Feder und von Reintitan für die Nut oder umgekehrt der Fall, da die Materialien unterschiedliche Härtegrade aufweisen.

Gemäß das Erfindung weist der Verbindungsträger mindestens eine Nut und die Platte mindestens eine zu der Nut komplementäre Feder zum Einsetzen in die Nut auf. Gemäß einer Ausgestaltung sind mehrere Nut-Feder-Verbindungen auf verschiedenen Seiten des Sensorsystems angeordnet, damit der Kraftfluß über das Sensorsystem erfolgt. Gemäß einer anderen Ausgestaltung ist die Nut kreisförmig oder ovalär und ist die Feder komplementär zu der Nut geformt und ist das Sensorsystem innerhalb der durch die Nut-Feder-Verbindung definierten, geschlossenen Kurve angeordnet. Bei dieser einzigen Nut-Feder-Verbindung kommt es ebenfalls zu einem Kraftfluß, durch das Sensorsystem. Gemäß einer Ausgestaltung ist auch die Platte kreisförmig oder ovalär, wobei die Form der Platte bevorzugt der Form der Nut-Feder-Verbindung entspricht.

Gemäß einer weiteren Ausgestaltung sind die Nut und die Feder konisch. Gemäß einer Ausgestaltung verjüngt sich die Nut zu ihrem Grund hin und/oder verjüngt sich die Feder zu ihrem freien Ende hin. Dies ermöglicht es, die Feder unter allmählichem Krafteinstieg in die Nut einzupressen und hierdurch eine besonders innige Verbindung zwischen Platte und Verbindungsträger herzustellen.

Gemäß einer Ausgestaltung ist die Platte aus einem härteren Material als der Verbindungsträger oder umgekehrt, so daß beim Herstellen der Nut-Feder-Verbindung eine besonders innige Verbindung zwischen Platte und Verbindungsträger durch Materialumformung erfolgt. Dadurch wird sichergestellt, daß die Übertragung der Kräfte dauerhaft gleichmäßig erfolgt.

Es ist möglich, Platte und Verbindungsträger allein durch eine Nut-Feder-Verbindung miteinander zu verbinden. Die Nut-Feder-Verbindung ist z.B. als Schnappverbindung ausgebildet oder zusätzliche Schnappelemente zum Verschnappen von Platte und Verbindungsträger kommen zur Nut-Feder-Verbindung hinzu. Gemäß einer Ausgestaltung ist mindestens eine Schraubverbindung zum Verbinden von Platte und Verbindungsträger vorhanden. Die Schraubverbindung ist z.B. zwischen dem Umfang einer kreisrunden Feder und einer kreisrunden Nut vorhanden. Gemäß einer Ausgestaltung umfaßt die Schraubverbindung eine Schraube, die durch ein Durchgangsloch der Platte in eine Gewindebohrung des Verbindungsträgers einschraubbar ist. Gemäß einer weiteren Ausgestaltung ist die Platte mittels mindestens zweier einander diametral gegenüberliegender Schraubverbindungen mit dem Verbindungsträger verbindbar. Dies ist z.B. vorteilhaft bei einer kreisringförmigen oder ovalären Nut-Feder-Verbindung zwischen Platte und Verbindungsträger bzw. einer entsprechend geformten Platte.

Es ist weiterhin möglich, die sogenannte separate Platte als Schraube mit Außengewinde herzustellen, welche innen zum Teil hohl ist, um den Dehnungsmeßstreifen und die notwendige Elektronik aufzunehmen.

Gemäß weiteren Ausgestaltungen ist die Verbindung zwischen dem Sensorsystem und der Platte durch Verklemmen, Festsetzen oder auf andere Weise erreicht. Ferner bezieht die Erfindung Ausgestaltungen ein, bei denen verschiedene Arten der Verbindung kombiniert sind.

Gemäß einer Ausgestaltung ist das Sensor- und/oder Telemetriesystem zumindest teilweise unterhalb der Platte in einem von der Nut-Feder- oder der Schraubverbindung umschlossenen Bereich angeordnet. Bei dieser Anordnung ist das Sensor- und/oder Telemetriesystem zumindest teilweise zwischen Verbindungsträger und Platte eingekapselt. Gemäß einer Ausgestaltung ist das Sensor- und/oder Telemetriesystem zumindest teilweise in einer auf der Oberseite der Platte angeordneten Kapsel angeordnet.

Gemäß einer weiteren Ausgestaltung ist die Kapsel aus Metall (z.B. aus Titan) und/oder Kunststoff. Es ist möglich, die Verkapselung durch eine Metallfolie vorzunehmen. Um sicherzustellen, daß das Signal nach außen gelangt, weist gemäß einer weiteren Ausgestaltung die metallische Kapsel ein Fenster auf, durch das das Telemetriesignal nach außen übertragen werden kann.

Gemäß einer Ausgestaltung weist das Sensor- und Telemetriesystem eine Antenne auf. Die Antenne begünstigt eine drahtlose Übertragung der Signale nach außen. Um die drahtlose Übertragung des Signals nach außen zu erleichtern, kann es günstig sein, die Sensoreinheit mit einer Antenne zu verbinden, deren Ende bis unter die Haut reicht. So kann die teilweise Absorption des Signals durch den Weichteilmantel umgangen werden.

Gemäß einer Ausgestaltung ist eine unter der Haut platzierbare Speicher- und Telemetrieeinheit mit einer in der Nähe des Sensor- und Telemetriesystems platzierbaren Empfängerantenne vorhanden. Hierdurch ist es möglich, den Teil des Sensor- und Telemetriesystems, der am Fixationssystem angeordnet ist, kleiner zu halten. Dadurch wird die Datenübertragung vereinfacht und der Energietransport erleichtert. Gemäß einer weiteren Ausgestaltung weist das Sensor- und Telemetriesystem eine Antenne zum drahtlosen Übertragen von Signalen an die Empfängerantenne auf.

Gemäß einer Ausgestaltung weist das Sensor- und Telemetriesystems und/oder die Speicher- und Telemetrieeinheit eine Energieversorgung durch Induktion und/oder durch mindestens ein Batteriesystem und/oder durch mindestens ein Akkusystem auf. Durch Induktion ist es möglich, von außen Energie einzuköppeln, die für die Durchführung von Messungen benötigt wird. Hierdurch kann auch eine Aufladung von Kondensatoren und/oder Batterien und/oder Akkus erfolgen.

Die Signale, die vom Sensor- und Telemetriesystem erhalten werden, können in einem externen Datenspeicher gesammelt werden, welcher mit bekannten Datenübermittlungssystemen und/oder Datenverarbeitungssystemen kommuniziert.

Das an einem Fixationssystem für Knochen während der Operation zum günstigsten Zeitpunkt montierbare Sensor- und Telemetriesystem erlaubt eine kontinuierliche Datenerfassung und eine drahtlose Datenübermittlung unter Verwendung handelsüblicher Speichertechnologien. Das System berücksichtigt ökonomische Aspekte und erlaubt den Einsatz, wenn die Indikation nachgewiesen ist.

Weitere Einzelheiten und Vorteile der Erfindung ergeben sich aus der nachfolgenden Beschreibung der anliegenden Zeichnungen von Ausführungsbeispielen. In den Zeichnungen zeigen:
- Fig. 1: Nicht-erfindungsgemäßes Fixationssystem mit Sensor- und Telemetriesystem auf einer Platte und multidirektional verblockbarer Knochenschraube in einer grobschematischen Perspektivansicht;
- Fig. 2a, b, c: Fixationssystem mit Sensor- und Telemetriesystem auf einer Platte in einer grobschematischen Perspektivansicht (Fig. 2a), in einem Längsschnitt durch den Verbindungsträger (Fig. 2b) und in einer Seitenansicht (Fig. 2c);
- Fig. 3a, b: Platte zum Tragen eines Sensors mit einer ovalären Feder in Unteransicht (Fig. 3a) und in einem Längsschnitt (Fig. 3b);
- Fig. 4: Platte gemäß Fig. 3 mit einem Verbindungsträger verschraubt in einem Längsschnitt, welcher auf der Oberseite eine verkapselte elektronische Einheit aufweist;
- Fig. 5: Nicht-erfindungsgemäße Separate Platte als aufschraubbares Element, welches auf der Innenseite den Dehnungsmeßstreifen aufweist;
- Fig. 6: Platte mit verkapseltem Sensor- und Telemetriesystem mit Antenne in einem Längsschnitt;
- Fig. 7: Platte mit verkapseltem Sensor- und Telemetriesystem in einer Kapsel mit Fenster für den Signalaustritt in einem Längsschnitt;
- Fig. 8: Platte mit Sensor- und Telemetriesystem in einer Kapsel mit Fenster und / oder einer Sendeantenne zur Erleichterung des Signalaustrittes sowie zugeordnete Speicher- und Telemetrieeinheit, welche sich unter der Haut befindet, in Seitenansicht;

Bei der nachfolgenden Erläuterung verschiedener Ausführungsbeispiele sind einander entsprechende Bauteile mit denselben Bezugsziffern bezeichnet.

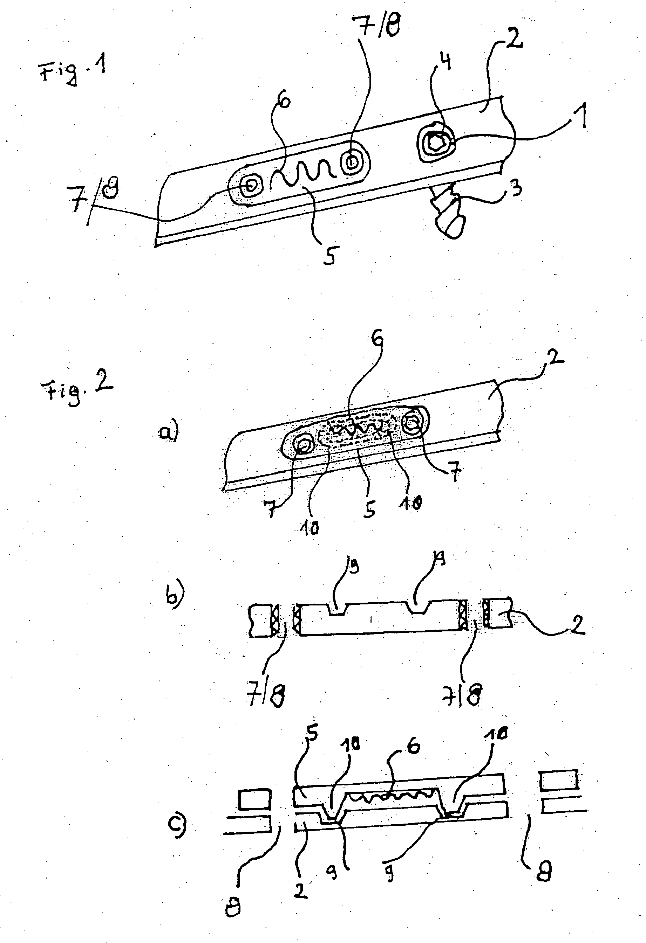

Gemäß Fig. 1 ist in einem Durchgangsloch 1 eines Verbindungsträgers 2 in Form einer Knochenplatte eine multidirektional verblockbare Knochenschraube 3 eingesetzt. Die Knochenschraube 3 ist mit einem Knochen verschraubbar, wobei von einem Gewinde in der Nähe des Schraubenkopfes 4 ein Gewinde in den Verbindungsträger 2 eingeformt wird.

Ferner ist auf dem Verbindungsträger 2 eine separate Platte 5 mit einem Dehnungsmeßstreifen 6 angeordnet. Die separate Platte 5 ist mittels weiterer Schrauben 7, die in Gewindelöcher 8 des Verbindungsträgers 2 eingedreht sind, mit dem Verbindungsträger 2 verbunden.

Gemäß Fig. 2 weist der Verbindungsträger 2 eine in der Draufsicht ovaläre und im Querschnitt konusförmige Nut 9 auf, in die eine komplementäre Feder 10 mit der separaten Platte 5 eingreift. Die separate Platte 5 ist wiederum mittels Schrauben 7, welche die Löcher der separaten Platte 5 durchgreifen und in Gewindelöcher 8 des Verbindungsträgers 2 eingreifen, mit dem Verbindungsträger 2 verschraubt. Hierdurch wird eine besonders innige Verbindung zwischen Platte 5 und Verbindungsträger 2 erzielt, so daß der Kraftfluß durch den Bereich zwischen den Nut-Feder-Verbindungen 9, 10 dem Kraftfluss innerhalb der ovalen Nut 9 des Verbindungsträgers 2 entspricht.

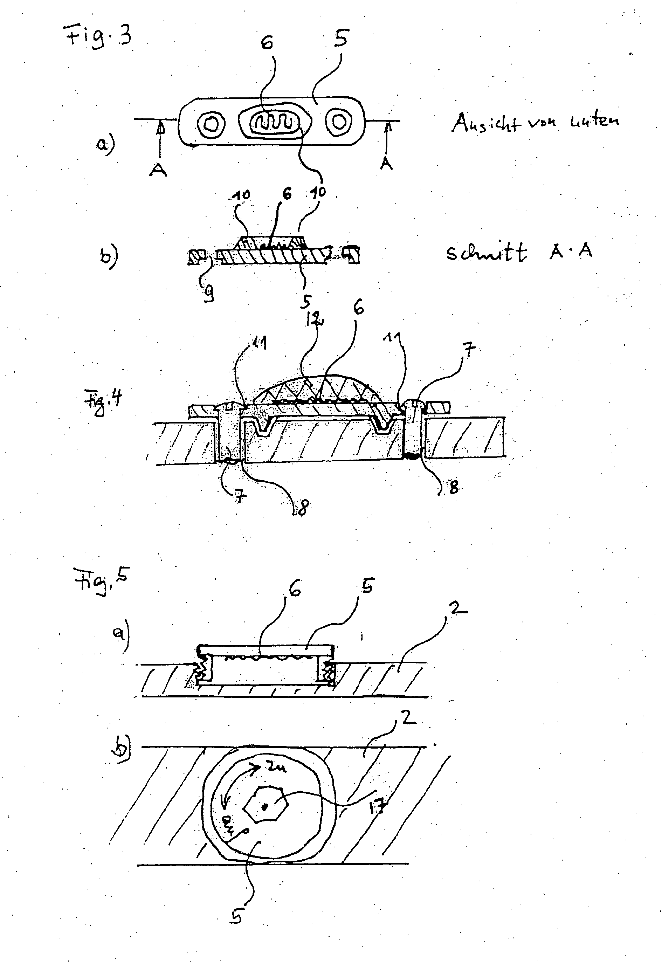

Gemäß Fig. 3 hat die separate Platte 5 eine ovaläre Feder 10 mit konischem Querschnitt. Die Feder 10 ist in einer komplementären Nut 9 des Verbindungsträgers 2 einfalzbar, wie in Fig. 4 gezeigt. Die separate Platte 5 ist mittels der Schrauben 7 fixiert, welche die Löcher 11 der separaten Platte 5 durchgreifen und in die Gewindelöcher 8 des Verbindungsträgers 2' eingeschraubt sind.

Gemäß Fig. 4 ist das Sensor- und Telemetriesystem 6 innerhalb einer Kapsel 12 auf der Oberseite der separaten Platte 5 angeordnet.

Gemäß Fig. 5 ist die separate Platte 5 als flache, innen teilweise hohle Schraube angelegt. Sie weist auf der Innenseite die Sensor- und Telemetrieeinheit 6 auf. Das Sensor- und Telemetriesystem 6 kann auch außen aufgebracht werden und ist in diesem Fall von einer Kapsel 12 umgeben. Das Eindrehen dieser einschraubbaren separaten Platte wird durch eine Sechskant-Ausnehmung 17 an der Außenfläche erleichtert.

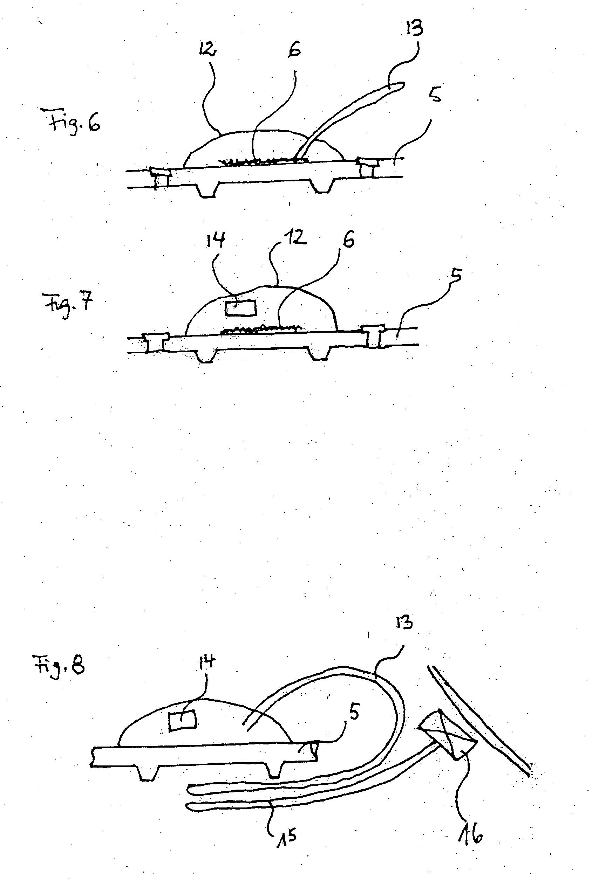

Gemäß Fig. 6 weist die separate Platte 5 ein Sensor- und Telemetriesystem 6 mit einer Kapsel 12 auf, wobei aus der Kapsel 12 eine Sendeantenne 13 herausgeführt ist, um eine erleichterte drahtlose Signalübertragung zu gewährleisten.

Gemäß Fig. 7 ist das Sensor- und Telemetriesystem 6 innerhalb einer Kapsel 12 auf der separaten Platte 5 angeordnet. Die Kapsel 12 weist ein Fenster 14 auf, so daß Signale des Sensor- und Telemetriesystems 6 aus der Kapsel 12 austreten können.

Gemäß Fig. 8 ist der separaten Platte 5 mit dem Fenster 14 eine weitere, z.B. unter der Haut liegende, Speicher- und Telemetrieeinheit 16 mit einer Empfängerantenne 15 zugeordnet. Die Speicher- und Telemetrieeinheit 16 befindet sich unter der Haut 18 und die Empfängerantenne 15 ist in der Nähe des Fensters 14 angeordnet, so daß eine drahtlose Signalübertragung durch das Fenster 14 und / oder die Sendeantenne 13 auf die Empfängerantenne 15 erfolgen kann. Energie kann der Speicher- und Telemetrieeinheit 16 von außen induktiv zugeführt werden oder die Speicher- und Telemetrieeinheit ist mit einer Batterie versehen, deren Speicherkapazität für die Dauer der Therapie ausreicht.

## Patentansprüche

1. Fixationssystem für Knochen mit einem Verbindungsträger (2), wenigstens einer in ein Durchgangsloch (1) des Verbindungsträgers (2) einsetzbaren Knochenschraube (3) und einem Sensor- und Telemetriesystem (6), wobei das Sensor- und Telemetriesystem (6) auf einer separaten Platte (5) angeordnet ist, die mit dem Verbindungsträger (2) verbindbar ist, **dadurch gekennzeichnet, daß** Verbindungsträger (2) und separate Platte (5) Einrichtungen zum Ausbilden mindestens einer Nut-Feder-Verbindung (9, 10) zwischen Verbindungsträger (2) und separater Platte (5) aufweisen.

2. Fixationssystem nach Anspruch 1, bei dem mindestens eine Knochenschraube (3) im Durchgangsloch (1) befestigbar ist.

3. Fixationssystem nach Anspruch 2, bei welchem der Knochenschraubenkopf (3) und /oder das Durchgangsloch (1) ein Gewinde zwischen Knochenschraube und Durchgangsloch aufweisen.

4. Fixationssystem nach Ansprüchen 1 bis 3, das Einrichtungen zum form- und/oder kraft- und/oder stoffschlüssigen dauerhaft festen Verbinden (9, 10) von Verbindungsträger (2) und separater Platte (5) aufweist.

5. Fixationssystem nach einem der Ansprüche 1 bis 4, bei dem das Sensorsystem (6) einen Dehnungsmeßstreifen umfaßt.

6. Fixationssystem nach Anspruch 5, bei dem der Dehnungsmeßstreifen (6) auf die separate Platte (5) aufgeklebt ist.

7. Fixationssystem nach Anspruch 6, bei dem die Einrichtungen zum Verbinden (9, 10) auf verschiedenen Seiten des Sensorsystems (6) angeordnet sind.

8. Fixationssystem nach einem der Ansprüche 1 bis 7, bei dem der Verbindungsträger (2) mindestens eine Nut (9) und die separate Platte (5) mindestens eine komplementäre Feder (10) zum Einsetzen in die Nut (9) aufweist.

9. Fixationssystem nach einem der Ansprüche 1 bis 8, bei dem mehrere Nut-Feder-Verbindungen (9, 10) auf verschiedenen Seiten des Sensorsystems (6) angeordnet sind.

10. Fixationssystem nach einem der Ansprüche 1 bis 9, bei dem die Nut (9) kreisförmig oder ovalär und die Feder (10') komplementär zu der Nut (9') geformt ist.

11. Fixationssystem nach einem der Ansprüche 1 bis 10, bei dem die separate Platte (5) kreisförmig oder ovalär ist.

12. Fixationssystem nach einem der Ansprüche 1 bis 11, bei dem die Nut (9) und die Feder (10) konisch sind.

13. Fixationssystem nach einem der Ansprüche 1 bis 12, bei dem die Nut (9) sich zu ihrem Grund hin verjüngt und/oder sich die Feder (10) zu ihrem freien Ende hin verjüngt.

14. Fixationssystem nach einem der Ansprüche 1 bis 13, bei dem die separate Platte (5) aus einem härteren Material ist als der Verbindungsträger (2) oder umgekehrt.

15. Fixationssystem nach einem der Ansprüche 1 bis 14 mit mindestens einer Schraubverbindung (7, 8) zum Verbinden von separater Platte (5) und Verbindungsträger (2).

16. Fixationssystem nach Anspruch 15, bei dem die Schraubverbindung (7, 8) eine Schraube umfaßt, die durch ein Durchgangsloch der separaten Platte (5) in eine Gewindebohrung des Verbindungsträgers (2) einschraubbar ist.

17. Fixationssystem nach Anspruch 15 oder 16, bei dem die separate Platte (5) mittels mindestens zwei einander diametral gegenüberliegender Schraubverbindungen (7, 8) mit dem Verbindungsträger (2) verbindbar ist.

18. Fixationssystem nach einem der Ansprüche 1 bis 17, bei dem das Sensor- und/oder Telemetriesystem (6) zumindest teilweise unterhalb der separaten Platte (5) in einem von der Nut-Feder-Verbindung (9, 10) umschlossenen Bereich angeordnet ist.

19. Fixationssystem nach einem der Ansprüche 1 bis 15, bei dem das Sensor- und/oder Telemetriesystem (6) zumindest teilweise in einer auf der Oberseite der separaten Platte (5) angeordneten Kapsel (12) angeordnet ist.

20. Fixationssystem nach Anspruch 19, bei dem die Kapsel (12) aus Metall oder Kunststoff besteht.

21. Fixationssystem nach einem der Ansprüche 1 bis 20, bei dem die separate Platte (5) als flache, innen hohle Schraube ausgestaltet ist, welche in ein Teilloch des Verbindungsträgers eingeschraubt wird.

22. Fixationssystem nach Anspruch 20 und 21, bei dem die metallische Kapsel (12) ein Fenster (14) aufweist.

23. Fixationssystem nach einem der Ansprüche 1 bis 22, bei dem das Sensor- und Telemetriesystem (6) eine Sendeantenne (13) aufweist.

24. Fixationssystem nach Anspruch 23, bei dem die Sendeantenne (13) innerhalb des subkutanen Gewebes platziert werden kann.

25. Fixationssystem nach Anspruch 23 oder 24, bei dem die Sendeantenne (13) eine Steckverbindung mit dem Sensor- und Telemetriesystem (6) aufweist.

26. Fixationssystem nach einem der Ansprüche 1 bis 25, das eine unter der Haut anordenbare Speicher- und Telemetrieeinheit (16) mit einer in der Nähe des Sensor- und Telemetriesystems (6) platzierbaren Empfängerantenne (15) umfaßt.

27. Fixationssystem nach einem der Ansprüche 1 bis 26, bei dem das Sensor- und Telemetriesystem (6) und/oder die Speicher- und Telemetrieeinheit (16) eine Energieversorgung durch Induktion und/oder durch mindestens ein Batteriesystem und/oder durch mindestens ein Akkusystem und/oder mindestens einen Kondensator aufweist.

28. Fixationssystem nach einem der Ansprüche 1 bis 27, bei dem der Verbindungsträger (2) eine Knochenplatte, ein Marknagel oder ein Fixateur externe ist.

## Claims

1. Fixation system for bones, comprising a connection support (2), at least one bone screw (3) insertable into a through-hole (1) of the connection support (2), and a sensor and telemetry system (6), wherein the sensor and telemetry system (6) is arranged on a separate plate (5) which is connectable to the connection support (2), **characterized in that** connection support (2) and separate plate (5) have means for forming at least one groove/tongue connection (9, 10) between connection support (2) and separate plate (5).

2. Fixation system according to Claim 1, in which at least one bone screw (3) can be secured in the through-hole (1).

3. Fixation system according to Claim 2, in which the head of the bone screw (3) and/or the through-hole (1) have a thread between bone screw and through-hole.

4. Fixation system according to Claims 1 to 3, having means for the form-fit and/or force-fit and/or cohesively bonded permanent connecting (9, 10) of connection support (2) and separate plate (5).

5. Fixation system according to one of Claims 1 to 4, in which the sensor system (6) comprises a strain gauge.

6. Fixation system according to Claim 5, in which the strain gauge (6) is adhesively bonded to the separate plate (5).

7. Fixation system according to Claim 6, in which the means of connection (9, 10) are arranged on different sides of the sensor system (6).

8. Fixation system according to one of Claims 1 to 7, in which the connection support (2) has at least one groove (9), and the separate plate (5) has at least one complementary tongue (10) for insertion into the groove (9).

9. Fixation system according to one of Claims 1 to 8, in which several groove/tongue connections (9, 10) are arranged on different sides of the sensor system (6).

10. Fixation system according to one of Claims 1 to 9, in which the groove (9) is circular or oval and the tongue (10') has a shape matching the groove (9').

11. Fixation system according to one of Claims 1 to 10, in which the separate plate (5) is circular or oval.

12. Fixation system according to one of Claims 1 to 11, in which the groove (9) and the tongue (10) are conical.

13. Fixation system according to one of Claims 1 to 12, in which the groove (9) narrows towards its base and/or the tongue (10) narrows towards its free end.

14. Fixation system according to one of Claims 1 to 13, in which the separate plate (5) is made of a harder material than the connection support (2), or vice versa.

15. Fixation system according to one of Claims 1 to 14, with at least one screw connection (7, 8) for connecting separate plate (5) and connection support (2).

16. Fixation system according to Claim 15, in which the screw connection (7, 8) comprises a screw which can be screwed through a through-hole of the separate plate (5) into a threaded bore of the connection support (2).

17. Fixation system according to Claim 15 or 16, in which the separate plate (5) is connectable to the connection support (2) by means of at least two diametrically opposite screw connections (7, 8).

18. Fixation system according to one of Claims 1 to 17, in which the sensor and/or telemetry system (6) is arranged at least partially below the separate plate (5) in an area surrounded by the groove/tongue connection (9, 10).

19. Fixation system according to one of Claims 1 to 15, in which the sensor and/or telemetry system (6) is arranged at least partially in a capsule (12) arranged on the top of the separate plate (5).

20. Fixation system according to Claim 19, in which the capsule (12) is made of metal or plastic.

21. Fixation system according to one of Claims 1 to 20, in which the separate plate (5) is designed as a flat screw which is hollow on the inside and which is screwed into a partial hole of the connection support.

22. Fixation system according to Claims 20 and 21, in which the metallic capsule (12) has a window (14).

23. Fixation system according to one of Claims 1 to 22, in which the sensor and telemetry system (6) has a transmitting antenna (13).

24. Fixation system according to Claim 23, in which the transmitting antenna (13) can be placed within the subcutaneous tissue.

25. Fixation system according to Claim 23 or 24, in which the transmitting antenna (13) has a plug connection to the sensor and telemetry system (6).

26. Fixation system according to one of Claims 1 to 25, which comprises a memory and telemetry unit (16) that can be arranged under the skin, with a receiving antenna (15) that can be placed near the sensor and telemetry system (6).

27. Fixation system according to one of Claims 1 to 26, in which the sensor and telemetry system (6) and/or the memory and telemetry unit (16) has a power supply through induction and/or through at least one battery system and/or through at least one rechargeable battery system and/or at least one capacitor.

28. Fixation system according to one of Claims 1 to 27, in which the connection support (2) is a bone plate, a medullary pin or an external fixator.

## Revendications

1. Système de fixation pour os, comportant un support d'assemblage (2), au moins une vis d'ostéosynthèse (3) à insérer dans un trou débouchant (1) du support d'assemblage (2), et un système de détection et de télémétrie (6), ledit système de détection et de télémétrie (6) étant disposé sur une plaquette (5) séparée, qui est destinée à être assemblée au support d'assemblage (2), **caractérisé en ce que** le support d'assemblage (2) et la plaquette (5) séparée comportent des dispositifs pour réaliser au moins un assemblage à rainure et languette (9, 10) entre le support d'assemblage (2) et la plaquette (5) séparée.

2. Système de fixation selon la revendication 1, dans lequel au moins une vis d'ostéosynthèse (3) peut être fixée dans le trou débouchant (1).

3. Système de fixation selon la revendication 2, dans lequel la tête de la vis d'ostéosynthèse (3) et/ou le trou débouchant (1) comportent un filetage entre la vis d'ostéosynthèse et le trou débouchant.

4. Système de fixation selon l'une quelconque des revendications 1 à 3, qui comporte des dispositifs pour un assemblage (9, 10) fixe durable, par conjugaison de forme et/ou par force et/ou par conjugaison de matière, entre le support d'assemblage (2) et la plaquette (5) séparée.

5. Système de fixation selon l'une quelconque des revendications 1 à 4, dans lequel le système de détection (6) comporte un extensomètre à fil d'acier.

6. Système de fixation selon la revendication 5, dans lequel l'extensomètre à fil d'acier (6) est collé sur la plaquette (5) séparée.

7. Système de fixation selon la revendication 6, dans lequel les dispositifs pour assembler (9, 10) sont disposés sur différentes faces du système de détection (6).

8. Système de fixation selon l'une quelconque des revendications 1 à 7, dans lequel le support d'assemblage (2) comporte au moins une rainure (9) et la plaquette (5) séparée comporte au moins une languette (10) complémentaire destinée à être insérée dans la rainure (9).

9. Système de fixation selon l'une quelconque des revendications 1 à 8, dans lequel plusieurs assemblages à rainure et languette (9, 10) sont disposés sur différentes faces du système de détection (6).

10. Système de fixation selon l'une quelconque des revendications 1 à 9, dans lequel la rainure (9) a une forme circulaire ou ovale et la languette (10') a une forme complémentaire à celle de la rainure (9).

11. Système de fixation selon l'une quelconque des revendications 1 à 10, dans lequel la plaquette (5) séparée est circulaire ou ovale.

12. Système de fixation selon l'une quelconque des revendications 1 à 11, dans lequel la rainure (9) et la languette (10) sont coniques.

13. Système de fixation selon l'une quelconque des revendications 1 à 12, dans lequel la rainure (9) se rétrécit vers le fond et/ou la languette (10) se rétrécit vers son extrémité libre.

14. Système de fixation selon l'une quelconque des revendications 1 à 13, dans lequel la plaquette (5) séparée est réalisée dans un matériau plus dur que celui du support d'assemblage (2) ou inversement.

15. Système de fixation selon l'une quelconque des revendications 1 à 14, comportant au moins un assemblage vissé (7, 8) pour assemble la plaquette (5) séparée et le support d'assemblage (2).

16. Système de fixation selon la revendication 15, dans lequel l'assemblage vissé (7, 8) comporte une vis, qui peut être vissée au travers du trou débouchant de la plaquette (5) séparée dans une forure filetée du support d'assemblage (2).

17. Système de fixation selon la revendication 15 ou 16, dans lequel la plaquette (5) séparée peut être assemblée au support d'assemblage (2) par l'intermédiaire d'au moins deux assemblages vissés (7, 8) diamétralement opposés l'un à l'autre.

18. Système de fixation selon l'une quelconque des revendications 1 à 17, dans lequel le système de détection et de télémétrie (6) est disposé au moins en partie au-dessous de la plaquette (5) séparée dans une zone entourée par l'assemblage à rainure et languette (9, 10).

19. Système de fixation selon l'une quelconque des revendications 1 à 15, dans lequel le système de détection et de télémétrie (6) est disposé au moins en partie dans une capsule (12) disposée sur la face supérieure de la plaquette (5) séparée.

20. Système de fixation selon la revendication 19, dans lequel la capsule (12) est réalisée en métal ou en matière plastique.

21. Système de fixation selon l'une quelconque des revendications 1 à 20, dans lequel la plaquette (5) séparée est réalisée sous la forme d'une vis plate creuse, qui est vissée dans un trou partiel du support d'assemblage.

22. Système de fixation selon les revendications 20 et 21, dans lequel la capsule (12) métallique comporte une fenêtre (14).

23. Système de fixation selon l'une quelconque des revendications 1 à 22, dans lequel le système de détection et de télémétrie (6) comporte une antenne d'émission (13).

24. Système de fixation selon la revendication 23, dans lequel l'antenne d'émission (13) peut être positionnée à l'intérieur du tissu subcutané.

25. Système de fixation selon la revendication 23 ou 24, dans lequel l'antenne d'émission (13) comporte un assemblage enfiché avec le système de détection et de télémétrie (6).

26. Système de fixation selon l'une quelconque des revendications 1 à 25, qui comporte une unité de mémoire et de télémétrie (16), à agencer sous la peau, avec une antenne de réception (15) à positionner dans le voisinage du système de détection et de télémétrie (6).

27. Système de fixation selon l'une quelconque des revendications 1 à 26, dans lequel le système de détection et de télémétrie (6) et/ou l'unité de mémoire et de télémétrie (16) comportent une alimentation en énergie par induction et/ou par au moins un système de batterie et/ou par au moins un système accumulateur et/ou au moins un condensateur.

28. Système de fixation selon l'une quelconque des revendications 1 à 27, dans lequel le support d'assemblage (2) est une plaque d'ostéosynthèse, un clou centromédullaire ou un fixateur externe.
